# EUROPEAN PATENT SPECIFICATION

(11) **EP 4 585 582 B1**
(45) Date of publication and mention of the grant of the patent: **25.11.2026**
(21) Application number: 24873859.3
(22) Date of filing: 04.07.2024
(51) Int. Cl.: C07C 2/06, C07C 7/04, C07C 11/02, C07C 11/107, B01J 19/00, C08J 3/02

(54) **METHOD FOR PROCESSING OF OLIGOMERIZATION REACTION BY-PRODUCTS**
VERFAHREN ZUR VERARBEITUNG VON NEBENPRODUKTEN EINER OLIGOMERISIERUNGSREAKTION
PROCÉDÉ DE TRAITEMENT DE SOUS-PRODUITS DE RÉACTION D'OLIGOMÉRISATION

(30) Priority: 09.11.2023 KR 20230154161
(43) Date of publication of application: 16.07.2025
(73) Proprietor: LG Chem, Ltd., Yeoui-daero, Youngdungpo-gu Seoul 07336 (KR)
(72) Inventor: BAEK, Se Won, Daejeon 34122 (KR); LEE, Jeong Seok, Daejeon 34122 (KR); SONG, Jong Hun, Daejeon 34122 (KR); HWANG, Moon Sub, Daejeon 34122 (KR); PAK, Geun Tae, Daejeon 34122 (KR)
(74) Representative: Hoffmann Eitle
(86) International application number: PCT/KR2024/009456
(87) International publication number: WO 2025/100677

(56) References cited:
- KR-A- 20150 006 067
- KR-A- 20150 051 190
- KR-A- 20160 099 478
- KR-B1- 102 266 927
- US-A1- 2023 183 148
- US-B1- 9 175 109
- US-B2- 8 658 750

## Description

### Technical Field

The present disclosure relates to a method for processing an oligomerization reaction by-product, and more particularly, to a method for processing an oligomerization reaction by-product which improves a pipe blockage problem by maximizing concentration of a polymer which is an oligomerization reaction by-product by a two-step concentration process to perform a treatment in a solidified state, and also, improves economic feasibility and stability by increasing a recovery rate of an ethylene oligomerization reaction product.

### [Background]

An α-olefin is an important material which is used in comonomers, cleaning agents, lubricants, plasticizers, and the like and is commercially widely used, and in particular, 1-hexene and 1-octene are often used as a comonomer for adjusting the density of polyethylene in preparation of linear low-density polyethylene (LLDPE).

The α-olefins such as 1-hexene and 1-octene are prepared representatively by an oligomerization reaction of ethylene. The ethylene oligomerization reaction is performed by an oligomerization reaction (trimerization reaction or tetramerization reaction) of ethylene in the presence of a catalyst by using ethylene as a reactant, and the reaction product produced by the reaction inevitably includes not only a multi-component hydrocarbon mixture including 1-hexene and 1-octene to be desired but also a polymer as a by-product during a catalytic reaction. The oligomerization reaction by-product floats in a liquid reaction medium in a reactor, and it may accumulate due to fouling in the reactor over time or be included in a reaction discharge and cause a problem in a post-process.

Therefore, the oligomerization reaction by-product included in the reaction discharge should be effectively discharged out of the process with appropriate product refinement. In this regard, conventionally, various methods for removing a fouled polymer inside the reactor by cleaning are disclosed, and since use of a solvent is needed in the cleaning process, there is a need to further separate the oligomerization reaction by-product by a refining process of recovering the solvent.

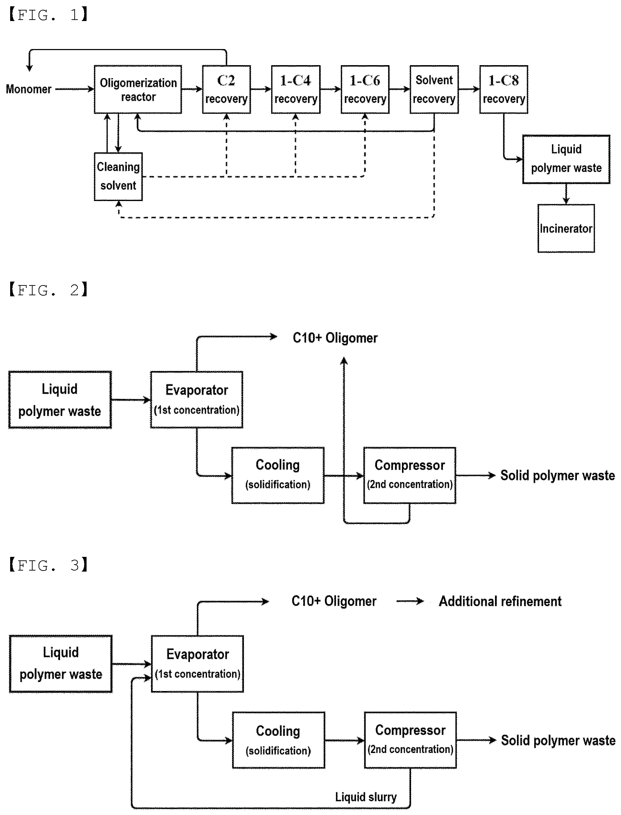

Meanwhile, conventionally, as shown in FIG. 1, a method of discharging the oligomerization reaction by-product separated by the refining process in a liquid phase and incinerating it was preferred. However, in order to discharge the liquid polymer as such, for preventing precipitation and coagulation of a polymer and maintaining flowability, discharge at a low concentration for maintaining a pipe temperature at or higher than an oligomer melting temperature or securing flowability and solubility is needed and loss of excessive product (or solvent) is involved. Since the operation method as such always involves a pipe blockage possibility due to local fouling in a commercial process, considering pipe complexity and nonuniformity depending on a large molecular weight distribution of the oligomerization reaction by-product, a more economical and also stable method of discharging and processing an oligomerization reaction by-product is needed.

### [Disclosure]

### [Technical Problem]

In order to solve the problems mentioned in the Background Art, an object of the present disclosure is to provide a method of increasing both economic feasibility and stability of a process by performing solidification through effective two-step concentration of a polymer which is a by-product produced in an oligomerization reaction (in particular, tetramerization reaction) of ethylene and discharging the polymer waste in a solid phase therethrough.

However, the object to be solved in the present disclosure is not limited to the object mentioned above, and other objects which are not mentioned may be clearly understood by a person skilled in the art from the following The present invention is defined in the appended descriptions. claims.

### [Technical Solution]

In one general aspect, a method for processing an oligomerization reaction by-product includes: in an oligomer preparation process of oligomerizing a monomer and a solvent to prepare a reaction product, introducing a liquid polymer waste which is the reaction product from which oligomers of lower than C10 and an unreacted monomer have been recovered to an evaporator, and then evaporating a fraction including C10 or higher oligomers (C10+ cut) to obtain a first concentrate including an oligomerization reaction by-product; introducing the first concentrate including the oligomerization reaction by-product to a cooling device to cool the first concentrate; and compressing the cooled first concentrate to obtain a second concentrated solid polymer waste.

### [Advantageous Effects]

According to the method for processing an oligomerization reaction by-product of the present disclosure, concentration of the oligomerization reaction by-product and a recovery rate of an ethylene oligomerization reaction product may be maximized.

According to the method for processing an oligomerization reaction by-product of the present disclosure, a polymer which is the oligomerization reaction by-product is solidified by two-step concentration, and a solid polymer waste is discharged, thereby minimizing process problems such as pipe blockage as compared with discharge of a liquid polymer waste.

An effect which may be obtained in the present disclosure is not limited to the effects mentioned above, and other effects which are not mentioned will be understood clearly by a person with ordinary skill in the art to which the present disclosure pertains from the following description.

### [Description of Drawings]

FIG. 1 is a flow chart of a process of general oligomerization preparation and polymer waste treatment.
FIG. 2 is a process flow chart of a method for processing an oligomerization reaction by-product according to an exemplary aspect of the present disclosure.
FIG. 3 is a process flow chart of a method for processing an oligomerization reaction by-product according to an exemplary aspect of the present disclosure.
FIG. 4 is a process flow chart of a method for processing an oligomerization reaction by-product according to an exemplary aspect of the present disclosure.
FIG. 5 is a GPC molecular weight distribution plot of a liquid polymer waste sample of Preparation Example 1.

### [Best Mode]

Regarding description of the drawings, similar reference numerals may be used for similar or related constituent elements.

A singular form of a noun corresponding to an item may include one or a plurality of items, unless otherwise explicitly stated in the relevant context.

In the present disclosure, each of the phrases such as "A or B", "at least one of A and B", "at least one of A or B", "A, B, or C", "at least one of A, B, and C", and "at least one of A, B, or C" may include any one of the items listed together with the corresponding phrase of the phrases or all possible combinations of them.

The term "and/or" includes a combination of a plurality of described related constituent elements or any one of a plurality of described related constituent elements.

The terms such as "1st" or "2nd", or "first" or "second" may be simply used for distinguishing a corresponding constituent element from other corresponding constituent elements, and the corresponding constituent elements are not limited in other aspects (e.g.: importance or order).

In addition, the terms such as "front surface", "back surface", "upper surface", "lower surface", "side surface", "left", "right", "upper", and "lower" used in the present disclosure are defined based on drawings, and the shape and position of each constituent element are not limited by the terms.

The term "comprises" or "have" is intended to specify the presence of stated features, steps, operations, constituent elements, parts, or a combination thereof, but do not preclude the presence or addition of one or more other features, numerals, steps, operations, constituent elements, parts, or a combination thereof.

When it is described that a constituent element is "connected to", "combined with", "supported on", or "in contact with" other constituent elements, it includes not only the case in which the constituent elements are directly connected, combined, supported, or in contact, but also the case in which they are indirectly connected, combined, supported, or in contact through a third constituent element.

When it is described that a constituent element is positioned "on" other constituent element, it includes not only the case in which the constituent element is in contact with another constituent element, but also the case in which another constituent element is present between the two constituent elements.

When unique manufacture and material allowable errors are suggested in the mentioned meaning, "about", "substantially", and the like used in the present disclosure are used in the meaning of the numerical value or in the meaning close to the numerical value, and are used to prevent the disclosure mentioning a correct or absolute numerical value order from being unfairly used by an unconscionable infringer for better understanding of the present disclosure.

The term "stream" used in the present disclosure may refer to a fluid flow in a process or may refer to a fluid itself flowing in a pipe. Specifically, the stream may refer to both a fluid itself flowing in a pipe connecting each device and a fluid flow. In addition, the fluid may include any one or more components of gas, liquid, and solid.

"Pressure" mentioned in the present disclosure refers to absolute pressure.

"Oligomers of lower than C10" mentioned in the present disclosure refer to a linear α-olefin having less than 10 carbon atoms, "C10 or higher oligomers" or "C10+" refers to a linear α-olefin having 10 or more carbon atoms, "C10 to C22 oligomers" refer to linear α-olefins having 10 to 22 carbon atoms, "C10 to C18 oligomers" refer to linear α-olefins having 10 to 18 carbon atoms, and "C10 to C16 oligomers" refer to linear α-olefins having 10 to 16 carbon atoms.

"Fraction" mentioned in the present disclosure refers to a component which may be extracted in a certain temperature range when distilling (fractioning) a liquid mixture.

Hereinafter, the present disclosure will be described in more detail referring to FIGS. 2 to 4, for better understanding of the present disclosure.

According to the present disclosure, a method for processing an oligomerization reaction by-product including: in an oligomer preparation process of oligomerizing a monomer and a solvent to prepare a reaction product, introducing a liquid polymer waste which is the reaction product from which oligomers of lower than C10 and an unreacted monomer have been recovered to an evaporator, and then evaporating a fraction including C10 or higher oligomers to obtain a first concentrate including an oligomerization reaction by-product; introducing the first concentrate including the oligomerization reaction by-product to a cooling device to cool the first concentrate; and compressing the cooled first concentrate to obtain a second concentrated solid polymer waste, is provided.

The present disclosure is a method for disposing a polymer including ultrahigh molecular weight polyethylene which is an oligomerization reaction by-product of an oligomerization reaction process having a difficulty in disposing a high-concentration liquid, specifically, a tetramerization reaction process, and may increase an recovery rate of a C10 or higher effective component from a liquid polymer waste after recovering a main product, facilitate a post-treatment of the polymer waste, and thus, increase economic feasibility of the entire process, by concentrating a polymer which is an oligomerization reaction by-product to the maximum and then processing the concentrate by solidification.

First, in the method for processing an oligomerization reaction by-product according to the present disclosure, a solution which is the reaction product from which oligomers of lower than C10 and an unreacted monomer have been recovered, that is, a liquid polymer waste is introduced to an evaporator, and then a fraction including C10 or higher oligomers is evaporated, thereby recovering a first concentrate including an oligomerization reaction by-product, as shown in FIG. 1 (first concentration step).

The liquid polymer waste introduced to the evaporator may be obtained by supplying a monomer stream and a solvent stream to the reactor to perform an oligomerization reaction to prepare a reaction product, and introducing a reactor discharge stream including the reaction product to a separation device to recover a fraction including the unreacted monomer and the oligomers of lower than C10.

The reactor may be for preparing an oligomer by oligomerizing a monomer in the presence of a catalyst and a solvent, and allows continuous preparation of an oligomer product.

In addition, the monomer may include ethylene. Specifically, a monomer stream including an ethylene monomer may be supplied to the reactor to prepare a linear α-olefin (LAO) which is the oligomer to be desired through an oligomerization reaction. Herein, the oligomerization reaction is performed in a reaction medium in a lower or a middle area of the reactor, and the oligomerization reaction of the monomer may be performed in a liquid state dissolved in a solvent in the presence of a catalyst and a cocatalyst. The oligomerization reaction may refer to a reaction in which a monomer is oligomerized. The oligomerization may be referred to as trimerization or tetramerization depending on the number of monomers to be polymerized, and these are collectively called multimerization. In the present disclosure, the oligomerization reaction may be a tetramerization reaction, and the main product of the tetramerization reaction is 1-octene (C8), but oligomers of lower than C8 and C10 or higher oligomers may be produced together. However, an amount of produced oligomer of higher than C22 may be insignificant.

The linear α-olefin, which is an important material used in comonomers, cleaning agents, lubricants, plasticizers, and the like, is commercially widely used, and in particular, 1-hexene (1-C6) and 1-octene (1-C8) are often used as a comonomer for adjusting the density of polyethylene in the preparation of linear low-density polyethylene (LLDPE). The linear α-olefin such as 1-hexene and 1-octene may be prepared by, for example, a trimerization reaction or tetramerization reaction of ethylene.

The oligomerization reaction of the monomer may be performed by a homogeneous liquid phase reaction, a slurry reaction in which the catalyst is in the form of being partially not dissolved or completely not dissolved, a two-phase liquid/liquid reaction, or a bulk phase reaction or gas phase reaction of which the product acts as a main medium, in the presence or absence of a solvent, by applying the reaction system and a common contact technology.

The catalyst may include a transition metal source. The transition metal source may be, for example, a compound including one or more selected from the group consisting of chromium (III) acetylacetonate, chromium (III) chloride tetrahydrofuran, chromium (III) 2-ethylhexanoate, chromium (III) tris(2,2,6,6-tetramethyl-3,5-heptanedionate), chromium (III) benzoylacetonate, chromium (III) hexafloro-2,4-pentanedionate, chromium (III) acetate hydroxide, chromium (III) acetate, chromium (III) butyrate, chromium (III) pentanoate, chromium (III) laurate, and chromium (III) stearate.

The cocatalyst may include, for example, one or more selected from the group consisting of trimethyl aluminum, triethyl aluminum, triisopropyl aluminum, triisobutyl aluminum, ethylaluminum sesquichloride, diethylaluminum chloride, ethyl aluminum dichloride, methylaluminoxane, modified methylaluminoxane, and borate.

As such, in the process of oligomerizing a monomer in the presence of a catalyst and a solvent, a polymer such as polyethylene may be produced as a by-product, in addition to an oligomer product. More specifically, the oligomerization reaction by-product may include ultrahigh molecular weight polyethylene (UHMWPE). Herein, the ultrahigh molecular weight polyethylene refers to polyethylene having a weight average molecular weight (Mw) of 1,000,000 g/mol or more, more specifically 1,000,000 g/mol to 3,000,000 g/mol. The molecular weight of 1,000,000 g/mol or more is a dominant physical property which affects evaporation process operation for processing the oligomerization reaction by-product. Specifically, ultrahigh molecular weight polyethylene having Mw of 1,000,000 g/mol or more may be precipitated or gel when a pipe temperature is lowered to a certain level or lower in a discharge process, resulting in process trouble. In addition, viscosity increases rapidly due to gelation when the high molecular liquid waste is evaporated, which makes evaporation process operation difficult, a concentration degree needs to be adjusted to a level where liquid operation is allowed during first concentration by evaporation.

The monomer stream may be supplied to the reactor through a monomer stream supply line provided in a lower portion of the reactor. Herein, the monomer may be supplied to the reactor in a gaseous state. Specifically, the monomer stream including a gaseous monomer may be supplied to the reactor, and the gaseous monomer may be dissolved in a solvent supplied to the reactor so that the oligomerization reaction may proceed in a liquid phase.

The monomer stream may be supplied from a naphtha cracking center (NCC). In the naphtha cracking center, a process including: introducing each of naphtha, C2 and C3 hydrocarbon compounds, propane, and the like to a supply raw material and performing cracking in each pyrolysis furnace; cooling cracking gas which has been pyrolyzed in each pyrolysis furnace to include hydrogen, and C1, C2, and C3 or higher hydrocarbon compounds; compressing the cooled cracking gas; and refining a cracking compression stream including hydrogen, and C1, C2, and C3 or higher hydrocarbon compounds, may be performed. Herein, the monomer stream may be a stream including an ethylene monomer (C2) separated from naphtha cracking.

The solvent stream may be supplied to the reactor through a solvent stream supply line provided in a lower side of the reactor. The solvent may include one or more selected from the group consisting of n-pentane, n-hexane, n-heptane, cyclohexane, methylcyclohexane, octane, cyclooctane, decane, dodecane, benzene, xylene, 1,3,5-trimethylbenzene, toluene, ethylbenzene, chlorobenzene, dichlorobenzene, and trichlorobenzene. The solvent may be used in combination of two or more, if necessary. Thus, a gaseous ethylene monomer may be liquefied at a higher temperature and a dissolution rate at which the gaseous ethylene monomer is dissolved in the solvent may be improved.

By-products produced in the oligomerization reaction in the reactor, for example, the polymer may be included at a content of 0.1 wt% to 5 wt%, specifically 0.1 wt% to 4 wt, and more specifically 0.1 wt% to 3 wt% based on 100 wt% of a reactor discharge solution which is the reactor discharge stream.

The reactor discharge stream may include a liquid first stream and a gaseous second stream. For example, in the reactor, a liquid reaction product including an oligomer product to be desired by the oligomerization reaction may be discharged through a product discharge line provided separately in a direction opposite to a lower side of the reactor, for example, a lower side of the reactor where the solvent supply line is formed, as the first stream. For example, the product discharge line may be formed at the same height as the solvent supply line. In addition, the product discharge line is connected to a separation device from the reactor and may transfer the reaction product to the separation device.

In addition, the second stream including a gaseous unreacted monomer which is not dissolved in a solvent and does not participate in the oligomerization reaction may be discharged through an unreacted monomer discharge line provided in an upper portion of the reactor. For example, the gaseous second stream may pass through a condenser to be condensed into a liquid phase, and be supplied to the separation device with the first stream. Herein, the first stream and the second stream may be supplied to the separation device through separate lines or may be joined as one line and supplied to the separation device.

According to an exemplary aspect of the present disclosure, the reactor discharge stream may be supplied to the separation device to separate a fraction including an unreacted monomer and oligomers of lower than C10 in the reaction product. The separation device may include a series of distillation columns. Specifically, as shown in FIG. 1, in the separation device, each fraction including the unreacted monomer and the oligomers of lower than C10 is separated from the reactor discharge stream including the supplied reaction product and recovered, and the unreacted monomer may be recycled to the reactor and reused. Herein, the upper discharge stream from the separation device may pass through a compressor and then circulated to the reactor in order to secure flowability to the reactor. In addition, the reaction product from which most of the fraction including the unreacted monomer and the oligomers of lower than C10 have been removed may be introduced to the evaporator as a liquid polymer waste.

According to an exemplary aspect of the present disclosure, the reaction product obtained by ethylene oligomerization is refined into a product through sequential distillation, and the oligomerization reaction by-product may be discharged in a liquid form with higher linear α-olefins of C10 or more as a lower discharge stream from a separation column for separating and recovering a C8 fraction which is a final main product.

As an example, as shown in FIG. 1, the reactor discharge stream is supplied to a separation device and is separated into a plurality of cuts and recovered. Specifically, the unreacted monomer (C2) in the reaction product is recovered, 1-butene (1-C4) is recovered, 1-hexene (1-C6) is recovered, the solvent is recovered, 1-octene (1-C8) is recovered, and then a remaining liquid polymer waste may be obtained. Herein, since flowability for liquid stream transfer should be maintained, the temperature needs to be maintained at or above a polymer melting temperature, and it is difficult to perform disposal by increasing a polymer concentration in the liquid polymer waste with the incineration method as shown in FIG. 1, which causes loss of a product or a solvent. In particular, since the oligomerization reaction by-product of the tetramerization reaction includes a large amount of the ultrahigh molecular weight polyethylene, it is vulnerable to the above problem, and thus, there is a limitation to additional evaporation application to the liquid polymer waste.

The liquid polymer waste remaining after recovering 1-octene (1-C8) introduced to the evaporator may include 0.1 wt% to 20 wt%, specifically 0.1 wt% to 10 wt% of the oligomerization reaction by-product, based on 100 wt% of the liquid polymer waste. In addition, the oligomerization reaction by-product may include an ultrahigh molecular weight polyethylene having a weight average molecular weight of 1,000,000 g/mol or more, and for example, may include 1 wt% to 50 wt%, specifically 1 wt% to 40 wt%, and more specifically 1 wt% to 30 wt% of the ultrahigh molecular weight polyethylene, based on 100 wt% of the oligomerization reaction by-product. The lower the content of the ultrahigh molecular weight polyethylene in the liquid polymer waste, the easier the concentration by evaporation.

The internal temperature of the evaporator may be a melting point of polyethylene or higher, and the internal pressure of the evaporator may be determined depending on the temperature. For example, the internal temperature of the evaporator may be 100°C to 250°C, specifically 130°C to 230°C, and more specifically 150°C to 220°C, and the internal pressure may be 1 torr to 760 torr, specifically 1 torr to 500 torr, and more specifically 1 torr to 300 torr. When the operating temperature and the pressure range of the evaporator are satisfied, the concentration rate of the oligomerization reaction by-product may be maximized, while precipitation of the oligomerization reaction by-product or gelation of the liquid polymer waste is prevented. Furthermore, when the internal temperature of the evaporator is too low, the oligomerization reaction by-product may not be melted, and when the internal temperature of the evaporator is too high, carbonization by action of other reaction by-products may occur.

In addition, the first concentrate may be in a high-viscosity solution state in a high temperature condition. The viscosity of the first concentrate may be increased as the concentration of the ultrahigh molecular weight polyethylene in the first concentrate is increased, and for example, the viscosity of the first concentrate at a temperature of about 190°C may be 70,000 cp or less, specifically 10,000 cp to 70,000 cp, and more specifically 30,000 cp to 70,000 cp. Since the high-viscosity liquid first concentrate as such may be gelled as the temperature is lowered, the temperature needs to be maintained at a certain degree in order to transfer the concentrate to a device of a subsequent process.

In addition, by performing an evaporation process in the temperature and pressure ranges in the evaporator, a fraction including C10 or higher oligomers (that is, heavy oligomers) which are higher linear α-olefins, specifically, a fraction including C10 to C22, more specifically C10 to C18 oligomers, may be evaporated to recover as the upper discharge stream from the evaporator. For example, in the tetramerization reaction, the amount of produced C22 or higher oligomers (higher linear α-olefin) may be insignificant.

A total solid content (TSC) of the oligomerization reaction by-product in the first concentrate by the evaporation may be 5 wt% to 50 wt%, specifically 10 wt% to 30 wt%, but is not limited thereto. When the total solid content of the oligomerization reaction by-product in the first concentrated solution by the evaporation is too high, viscosity increases rapidly, so that evaporation operation of first concentration becomes difficult or a process may cause a problem by the gelation. When the total solid content of the oligomerization reaction by-product in the first concentrate by the evaporation is too low, economic feasibility of evaporation operation is lowered, and subsequent stable operation of a cooling process and a compression process may be difficult.

In addition, a recovery rate of C10 or higher oligomers which are recovered by the first concentration may be adjusted depending on the solid content of the oligomerization reaction by-product introduced to the evaporator before the evaporation process and the solid content of the oligomerization reaction by-product after the evaporation process. For example, the recovery rate of C10 or higher oligomers by the first concentration is preferably at least 30 wt% to 90 wt%, specifically 50 wt% to 80 wt%, based on the total amount of C10 or higher oligomers introduced to the evaporator. When the recovery rate is satisfied, the evaporation operation of the first concentration is economical and stable operation is allowed.

Subsequently, referring to FIG. 2, the first concentrate including the oligomerization reaction by-product may be cooled and gelled (solidification step).

The cooling step may include introducing a liquid high-viscosity first concentrate including the oligomerization reaction by-product to a cooling device and cooling the first concentrate to convert the concentrate into a gel phase which is a semi-solid state, and crushing the first concentrate of a gel phase, and if necessary, further cooling may be performed after the crushing. More specifically, the liquid first concentrate including the oligomerization reaction by-product which is concentrated in the evaporator may be transferred to a cooling device equipped with a heat exchanger by a pump, gelled, that is, solidified by the cooling process in the cooling device, and then crushed while being discharged through a die-plate mounted in a rear state of the cooling device after solidification.

The cooling device may include a melt cooler, a screw conveyor cooler equipped with a cooling jacket, a cooling tank equipped with a cooling jacket and a crushing stirrer, or the like. Herein, the melt cooler may be a multi-tube type heat exchanger including a double tube or a cooling shell equipped with a cooling jacket.

As an example, when the melt cooler is used as the cooling device, the liquid first concentrate including the oligomerization reaction by-product may be first cooled by heat exchange through a melt cooler to form a gel phase, and the gel phase may pass through a die-plate mounted in the rear stage of the melt cooler to crush the gel phase, which is then second cooled. In addition, during cooling using the melt cooler, the first concentrate may be cooled to a low temperature of 80°C or lower. For example, the cooling temperature of the first concentrate may be 10°C to 80°C, specifically 20°C to 70°C, and more specifically 30°C to 60°C. By cooling a polymer waste to 80°C or lower during the solidification, polymer re-melting may be prevented in the compression process described later. Herein, cooling rates of the first cooling and the second cooling may be 1°C/min to 30°C/min, specifically 1°C/min to 20°C/min, respectively, but are not limited thereto.

As another example, when a screw conveyor cooler equipped with a cooling jacket is used as the cooling device, the first concentrate at a high temperature introduced to an inlet of the screw conveyor cooler is slowly gelled (solidified) while being transferred to a discharge part through a rotary screw, and passes through the die-plate installed in the discharge part to obtain a crushed product of a gel phase. Herein, a cooling rate may be 1°C/min to 30°C/min, specifically 1°C/min to 20°C/min, respectively, but is not limited thereto.

As another example, when a cooling tank equipped with a cooling jacket and a crushing stirrer is used as the cooling device, a liquid first concentrate at a high temperature is introduced to the cooling tank and then cooled to 80°C or lower without stirring to form a gel phase, and the gelled first concentrate is crushed by stirring. For example, the cooling temperature of the first concentrate may be 10°C to 80°C, specifically 20°C to 70°C, and more specifically 30°C to 60°C. When the stirring is performed before the cooling temperature of the liquid first concentrate reached to the above range, strong flocculation causes a problem in the operation of the cooling tank, and thus, the gel phase needs to be crushed by stirring after sufficient cooling. Herein, a cooling rate may be 1°C/min to 30°C/min, specifically 1°C/min to 20°C/min, respectively, but is not limited thereto.

Subsequently, referring to FIG. 2, the cooled first concentrate may be compressed to obtain a second concentrated solid polymer waste (second cooling step).

The compression, which is a physical second concentration method, may use a commonly known compressor without a limitation, and if necessary, may be performed once or more. The concentration of the reaction by-product and the recovery rate of the reaction product may be maximized by the second concentration. In addition, a process problem such as pipe blockage which is problematic during liquid discharge of the polymer waste may be solved by molded solidified discharge.

In addition, the temperature of the first concentrate gelled in the compression may be 10°C to 80°C, specifically 20°C to 70°C, and more specifically 30°C to 60°C. When the compression process is performed within the temperature range, polymer re-melting may be prevented.

The total solid content (TSC) of the oligomerization reaction by-product in the second concentrated solid polymer waste by the compression may be 25 wt% to 95 wt%, specifically 25 wt% to 60 wt%, and more specifically 25 wt% to 50 wt%, but is not limited thereto.

In addition, a fraction including C10 or higher oligomers may be further recovered by the second concentration. The final recovery rate of the C10 or higher oligomers by the first concentration and second concentration may be 80 wt% to 99 wt%, specifically 83 wt% to 95 wt%, based on the total amount of C10 or higher oligomers introduced to the evaporator.

More specifically, in the second concentration step, a liquid slurry including C10 or higher oligomers, specifically C10 to C22 oligomers may be recovered by the compression. However, since some polyethylene (PE) solids may be included in the liquid slurry and leak out due to the limitation of physical compression, it is preferred to perform an additional refinement or separation process for removing an oligomerization reaction by-product incorporated into a liquid slurry and recovering C10 or higher oligomers.

For example, as shown in FIG. 3, the liquid slurry may be recirculated to the evaporator. Thus, a fraction including C10 or higher oligomers, specifically a fraction including C10 to C22 oligomers, and more specifically a fraction including C10 to C18 oligomers, are further recovered, and a polyethylene effluent may be further removed.

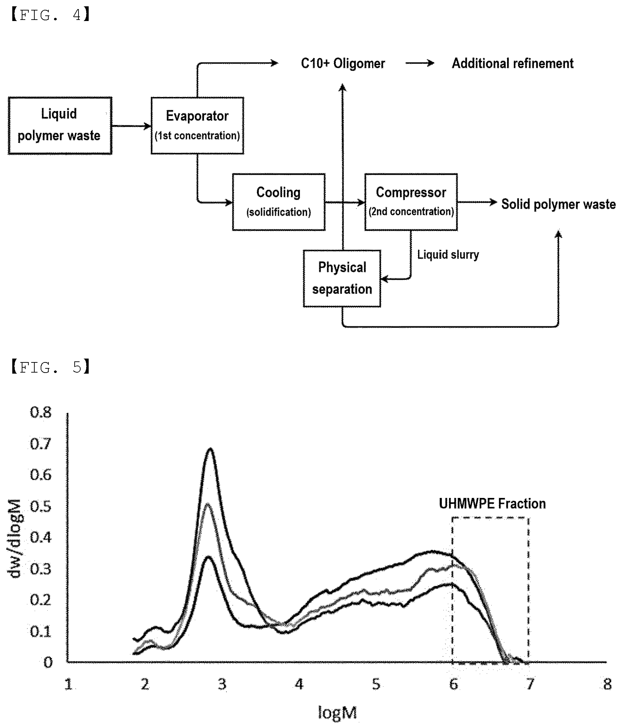

For example, as shown in FIG. 4, a fraction including C10 or higher oligomers, specifically a fraction including C10 to C22 oligomers, and more specifically a fraction including C10 to C18 oligomers may be recovered by performing physical separation on the liquid slurry. The polyethylene effluent incorporated in the liquid slurry is removed by physical separation, and then the fraction including C10 or higher oligomers may be recovered. The physical separation may be performed using one or more devices selected from a filter, a decanter, and a centrifuge, but is not limited thereto.

Meanwhile, the fraction including C10 or higher oligomers recovered by the method for processing an oligomerization reaction by-product according to the present disclosure may be commercialized by an additional refinement process, thereby further increasing economic feasibility.

According to an exemplary aspect of the present disclosure, during oligomer manufacture and reaction by-product treatment, if necessary, a device required for manufacturing an oligomer and removing an oligomerization reaction by-product such as a valve, a condenser, a reboiler, a pump, a separation device, a compressor, and a mixer may be further installed.

Hereinabove, the method for processing an oligomerization reaction by-product according to the present disclosure has been described and illustrated in the drawings, but the description and the illustration in the drawings are the description and the illustration of only core constitutions for understanding of the present disclosure, and in addition to the process and apparatus described above and illustrated in the drawings, the process and the apparatus which are not described and illustrated separately may be appropriately applied and used for performing the method for processing an oligomerization reaction by-product according to the present disclosure.

Hereinafter, the present disclosure will be described in detail through the following examples. However, the following examples are for describing the present disclosure in more detail, and the scope of the present disclosure is not limited to the following examples.

### [Examples]

### Preparation Example: preparation of oligomerization reaction by-product sample by ethylene tetramerization reaction and molecular weight distribution analysis thereof

Referring to FIG. 1, an ethylene tetramerization reaction was performed at a temperature of 60°C under conditions of a pressure of 31 bar in a pilot scale reactor, and after a continuous reaction for 70 hours, the reaction solution in the reactor was drained and filtered, thereby obtaining a liquid polymer waste (waste polymer solution) sample. At this time, three types of samples were obtained at different times and positions, and the composition range of the liquid polymer waste was calculated by quantitative analysis of the obtained liquid polymer waste sample and the results of process simulation of FIG. 1, and is shown in the following Table 1.

More specifically, in the following Table 1, the composition of the reactor discharge stream, and the composition of the liquid polymer waste obtained from the reactor discharge stream when the polymer content as the oligomerization reaction by-product was 0.1 wt%, 0.3 wt%, and 0.5 wt%, respectively (PE 0.1, PE 0.3, and PE 0.5) are shown.

**[Table 1]**

| Classification | Composition of reactor discharge stream (wt%) | Composition of polymer waste (wt%) | | |
|---|---|---|---|---|
| | | PE 0.1 | PE 0.3 | PE 0.5 |
| Nitrogen | 0.02 | 0 | 0 | 0 |
| Methane | 0.03 | 0 | 0 | 0 |
| Ethylene | 10.11 | 0 | 0 | 0 |
| Ethane | 0.49 | 0 | 0 | 0 |
| C4 fraction | 4.52 | 0 | 0 | 0 |
| C6 fraction | 8.28 | 0 | 0 | 0 |
| C8 fraction | 17.91 | 0 | 0 | 0 |
| C10 fraction | 0.62 | 13.21 | 12.67 | 12.17 |
| C12 fraction | 1.75 | 37.54 | 36 | 34.58 |
| C14 fraction | 1.21 | 25.91 | 24.85 | 23.87 |
| C16 fraction | 0.29 | 6.19 | 5.94 | 5.7 |
| C18 fraction | 0.35 | 7.45 | 7.15 | 6.86 |
| C20 fraction | 0.21 | 4.51 | 4.33 | 4.16 |
| C22+ fraction | 0.14 | 3.04 | 2.92 | 2.8 |
| **Polymer (PE)** | **0.1-0.5** | **2.14** | **6.16** | **9.86** |
| Solvent | 53.77 | 0 | 0 | 0 |
| Total | 100 | 100 | 100 | 100 |

In addition, the molecular weight distribution of the liquid polymer waste sample is as shown in FIG. 5. Referring to FIG. 5 and Table 1, it was confirmed that an oligomerization reaction by-product having a broad molecular weight distribution from a wax component to ultrahigh molecular weight polyethylene (UHMWPE) during a tetramerization reaction was produced. That is, it was confirmed from the present experiment that ultrahigh molecular weight polyethylene having a weight average molecular weight (Mw) of 1,000,000 g/mol or more was produced in the tetramerization reaction. In addition, the content of UHMWPE in the oligomerization reaction by-product had a weight ratio of about 10 to 40 by separation by molecular weight by a solvent extraction method.

### Experimental Example 1: measurement of solubility and precipitation temperature of oligomerization reaction by-product

A fraction having Mw of 1,000,000 g/mol or more is a dominant physical property which affects an evaporation process operation, and specifically, since ultrahigh molecular weight polyethylene having Mw of 1,000,000 or more causes process trouble due to precipitation and gelation during liquid polymer waste discharge, and also rapidly increases viscosity due to gelation during evaporation of the polymer liquid waste to make the evaporation process operation difficult, confirmation of a polymer solution concentration to a level to allow operation even during concentration is needed.

Thus, in the present experiment, in order to confirm the solution concentration and temperature conditions of the oligomerization reaction by-product which may be discharged as a liquid phase, the liquid polymer waste sample (PE 0.3, UHMWPE weight ratio of about 40 wt%) obtained in the preparation example and C14 (1-tetradecene) as a representative solvent of a higher linear α-olefin were selected and solubility for each polymer concentration was measured.

Specifically, the total solution composition was set to 200 **g,** the liquid polymer waste sample was increased, and the solution for each concentration was prepared. In order to uniformly dissolve the polymer waste in the solvent, the temperature was maintained at 200°C for 3 hours, the temperature of the dissolution tank was slowly cooled at - 1°C/min with stirring, the temperature at which polymer precipitation started or gelation occurred was recorded, and the results are shown in the following Table 2.

**[Table 2]**

| Solution concentration | Precipitation start temperature | Precipitation/gelation |
|---|---|---|
| 2 wt% | 107°C | Precipitation |
| 4 wt% | 112 °C | Precipitation |
| 6 wt% | 125°C | Precipitation |
| 14 wt% | 145°C | Precipitation |
| 18 wt% | 200°C | Gelation |
| 25 wt% | 200°C | Not soluble within 3 hours, gel phase |

Referring to Table 2, when the polymer concentration was the lowest (2 wt%), polymer precipitation started from 107°C, the precipitation start temperature rose until the concentration was 14 wt% as the polymer concentration was increased, the polymer was all dissolved at 200°C from a concentration of 18 wt% or more and then was in a gelation state, and the polymer was not dissolved within 3 hours at 200°C at a higher concentration of 25 wt%.

That is, even in a low concentration conditions, a temperature condition of at least about 110°C or higher was needed for preventing precipitation of a liquid polymer waste, and also, it was confirmed that a concentration at which the polymer may be dissolved without precipitation when a pipe temperature was maintained at about 150°C or higher was about 14 wt%, and a high temperature condition of 200°C or higher was needed for stable disposal of the liquid polymer waste having a concentration of 14 wt% or more.

### Example 1

As described in the preparation example, ethylene and a solvent were introduced to a pilot scale reactor, and an ethylene tetramerization (oligomerization) reaction was performed at a temperature of 60°C and a pressure condition of 31 bar. After the oligomerization reaction, the reactor discharge stream was introduced to the separation column to separate and recover unreacted ethylene and a C8 or lower fraction (oligomer), and a liquid polymer waste from which the C8 or lower fraction had been removed was obtained as the lower discharge stream from the separation column. At this time, the total solid content (TSC) of the oligomerization reaction by-product in the liquid polymer waste was 6.3 wt%.

First, as shown in FIG. 2, the liquid polymer waste having TSC of 6.3 wt% was introduced to the evaporator, which was operated at 165°C at a pressure of 50 torr until distillation recovery of the oligomer stopped to perform first concentration, and the first concentrated liquid polymer waste at this time was concentrated to TSC of 14.4 wt%. At this time, the viscosity of the first concentrated solution was 30,000 cp (190°C, shear rate: 0.1 s⁻¹). Meanwhile, the C10 or higher fraction (oligomer) which was a higher linear α-olefin in the liquid polymer waste was evaporated and recovered as the upper discharge stream of the evaporator.

Subsequently, the first concentrated liquid polymer waste was drained in a cooling tank equipped with a cooling jacket, cooled to 30°C at a cooling rate of 4°C/min to gelate the waste, and crushed to obtain a solidified product.

Subsequently, the product was compressed and recovered with an expeller for second concentration of the solidified product to obtain a solid polymer waste concentrated to TSC of 28.3 wt%.

### Example 2

As shown in Example 1, the liquid polymer waste having TSC of 6.3 wt% was introduced to the evaporator, which was operated at 180°C at a pressure of 100 torr until distillation recovery of the oligomer stopped to perform first concentration, and the first concentrated liquid polymer waste at this time was concentrated to TSC of 27.2 wt%. At this time, the viscosity of the first concentrated solution was 70,000 cp (190°C, shear rate: 0.1 s⁻¹), resulting in stopping of the operation of the stirrer of the evaporator. Meanwhile, the C10 or higher fraction (oligomer) which was a higher linear α-olefin in the liquid polymer waste was evaporated and recovered as the upper discharge stream of the evaporator.

Subsequently, the first concentrate was drained in a cooling tank equipped with a cooling jacket, cooled to 30°C at a cooling rate of 4°C/min, and crushed to obtain a solidified product.

Subsequently, the solidified product was compressed and recovered with an expeller to obtain a solid polymer waste concentrated to TSC of 44.2 wt%.

### Example 3

The solid polymer waste having TSC of 28 wt% obtained by performing first concentration and second concentration in Example 1 was compressed once more (compression twice) with the same compressor, and as a result, a solid polymer waste concentrated to TSC of 45.7 wt% was obtained.

### Comparative Example 1

The process was performed in the same manner as in Example 1, except that solidification and second concentration processes were not performed after concentrating the liquid polymer waste by the evaporator.

### Comparative Example 2

The process was performed in the same manner as in Example 2, except that solidification and second concentration processes were not performed after concentrating the liquid polymer waste by the evaporator.

### Comparative Example 3

The process was performed in the same manner as in Example 1, except that the liquid polymer waste having TSC of 6.3 wt% was directly cooled without first concentration by the evaporator and then a direct compression process was performed.

### Reference Example 1

A liquid polymer waste having TSC of 6.3 wt% including only a polyethylene wax component (weight average molecular weight: 3,000 g/mol) without a UHMWPE fraction as an oligomerization reaction by-product was prepared and concentrated by the evaporator. More specifically, concentration was performed by operation by the evaporator at 165°C at a pressure of 50 torr until the distillation recovery of the oligomer stopped. At this time, the viscosity of the concentrated solution was 200 cp (190°C, shear rate: 0.1 s⁻¹).

### Experimental Example 2

The process conditions, the final state of the polymer wastes therefrom, the total solid contents (TSC, wt%), and the recovery rates (wt%) of C10 or higher oligomers of Examples 1 and 2 and Comparative Examples 1 and 2 are shown in the following Table 3.

In the following Table 3, "presence or absence of UHMWPE" represents whether the ultrahigh molecular weight polyethylene was included in the oligomerization reaction by-product in the liquid polymer waste, and "final state of polymer waste" represents the phase and the temperature of the final polymer waste after the concentration process. For reference, the weight ratio of UHMWPE in the oligomerization reaction by-product when the ultrahigh molecular weight polyethylene was included was about 40 wt%.

In addition, "TSC (wt%)" represents the total solid content of the oligomerization reaction by-product in the final polymer waste, and "C10+ recovery rate (wt%)" is a recovery rate (wt%) of C10 or higher oligomers recovered throughout the process based on 100 wt% of C10 or higher oligomers in the liquid polymer waste having TSC of 6.3 wt%.

**[Table 3]**

| Classificat -ion | Presence or absence of UHMWPE | Evaporator (first concentration) | | Compressor (second concentration) | | Final state of polymer waste | C10+ recovery rate (wt%) |
|---|---|---|---|---|---|---|---|
| | | Operation conditions | TSC (wt%) | Number of campressions | TSC (wt%) | | |
| Example 1 | ○ | 165°C, 50 torr | 14.4 | 1 | 28.3 | Solid phase (room tenperature) | 83.0 |
| Example 2 | ○ | 180°C, 100 torr | 27.2 | 1 | 44.2 | Solid phase (room tenperature) | 91.5 |
| Example 3 | ○ | 165°C, 50 torr | 14.4 | 2 | 45.7 | Solid phase (room tenperature) | 92.5 |
| Comparative Example 1 | ○ | 165°C, 50 torr | 14.4 | × | - | High viscosity liquid phase (165°C) | 60.0 |
| Comparative Example 2 | ○ | 180°C, 100 torr | 27.2 | × | - | High viscosity liquid phase (180°C) | 82.0 |
| Comparative Example 3 | ○ | × | - | 1 | Not compr essib le | Slurry phase (room temperature) | Not collecta ble |
| Reference Example 1 | × | 165°C, 50 torr | 55.8 | × | - | Low-viscosity liquid phase (80°C) | 94.6 |

Referring to Table 3, since Examples 1 to 3 in which the liquid polymer waste including UHMWPE was evaporated and then compressed to perform second concentration had a higher polymer solid content in the finally discharge waste than that of Comparative Examples 1 and 2 in which the second concentration was not performed, the oligomerization reaction by-product was able to be effectively removed, and thus, the oligomer recovery rate of C10 or higher which is a higher linear α-olefin was improved. In addition, the polymer waste was finally discharged in a solid phase, thereby preventing problems such as pipe blockage due to the oligomerization reaction by-product.

More specifically, referring to Comparative Example 2, when the liquid polymer waste including UHMWPE was evaporated, further concentration was difficult only with the evaporation due to rapid viscosity increase at TSC of 27 wt%, and thus, there was a limitation to the recovery rate of C10 or higher oligomers. Besides, as the concentration was performed to a higher concentration, process problems such as pipe blockage during discharge of the polymer waste occurred due to the high viscosity and the high gelation temperature.

In this regard, in Reference Example 1 in which concentration of the liquid polymer waste which did not include UHMWPE was experimented, high concentration to TSC of 55.8 wt% was allowed only with the evaporation process which is the first concentration, and the polymer solution concentrated at this time did not gelate even at a low temperature of 80°C and maintained a stable low-viscosity liquid phase. This is because the UHMWPE component was absent in the oligomerization reaction by-product, or if present at a low ratio, high concentration of the polymer waste was allowed only with the first evaporation operation to increase the recovery rate of C10 or higher oligomers, and furthermore, the liquid phase was able to be disposed well at a temperature maintained at 80°C or higher even when the polymer waste was in a highly concentrated state.

Meanwhile, in Comparative Example 3, the liquid polymer waste having TSC of 6.3 wt% was directly cooled without evaporation and concentration, and at this time, it was cooled in a slurry form which was a mixture of an oligomer solution and some gels. In addition, when it was introduced to a compressor, the polymer solution phase flowed out through the compressor liquid discharge port to prevent smooth compression. As such, when the solution at a low temperature was cooled and compressed without first concentration, it was confirmed that precipitation occurred in the cooling step and it is difficult to compression by the compressor. That is, in order to compression by physical concentration, the polymer solution needs to be concentrated to TSC of about 10 wt% or more as in Examples 1 to 3.

Hereinabove, the exemplary aspects of the present disclosure have been described, but the present disclosure is not limited thereto, and those with ordinary skill in the art will understand that various changes and modification are possible within the range of the appended claims.

## Claims

1. A method for processing an oligomerization reaction by-product, comprising:
in an oligomer preparation process of oligomerizing a monomer and a solvent to prepare a reaction product,
introducing a liquid polymer waste to an evaporator, wherein the liquid polymer waste is a residue remaining after recovering oligomers of lower than C10 and an unreacted monomer from the reaction product, and then evaporating a fraction comprising C10 or higher oligomers to obtain a first concentrate comprising an oligomerization reaction by-product;
cooling the first concentrate comprising the oligomerization reaction by-products by introducing it into a cooling device; and
compressing the cooled first concentrate to obtain a second concentrated solid polymer waste.

2. The method of claim 1,
wherein in the evaporator, a fraction comprising C10 to C22 oligomers is evaporated and recovered as an upper discharge stream from the evaporator.

3. The method of claim 1, wherein in the second concentration, a liquid slurry comprising C10 to C22 oligomers is recovered by the compression.

4. The method of claim 3, further comprising:
recirculating the liquid slurry to the evaporator.

5. The method of claim 3, comprising:
recovering the fraction comprising the C10 to C22 oligomers by performing physical separation on the liquid slurry using one or more devices selected from a filter, a decanter, and a centrifuge.

6. The method of claim 1,
wherein the monomer includes ethylene, and
the oligomers include a linear α-olefin.

7. The method of claim 1,
wherein the oligomerization reaction by-product includes ultrahigh molecular weight polyethylene, and
the ultrahigh molecular weight polyethylene has a weight average molecular weight (Mw) of 1,000,000 g/mol or more.

8. The method of claim 7,
wherein the oligomerization reaction by-product is included at 0.1 wt% to 20 wt% based on 100 wt% of the liquid polymer waste introduced to the evaporator, and
the ultrahigh molecular weight polyethylene is included at 1 wt% to 50 wt% based on 100 wt% of the oligomerization reaction by-product.

9. The method of claim 1,
wherein a total solid content (TSC) of the oligomerization reaction by-product in the first concentrate is 10 wt% to 30 wt%, and
a total solid content (TSC) of the oligomerization reaction by-product in the second concentrated solid polymer waste is 25 wt% to 95 wt%.

10. The method of claim 1,
wherein the liquid polymer waste introduced to the evaporator is obtained by supplying a monomer stream and a solvent stream to a reactor to perform an oligomerization reaction to prepare the reaction product, and introducing a reactor discharge stream including the reaction product to a separation device to recover a fraction including the unreacted monomer and the oligomers of lower than C10.

11. The method claim 1,
wherein an internal temperature of the evaporator is from 100°C to 250°, and an internal pressure of the evaporator is from 1 torr to 760 torr.

12. The method of claim 1,
wherein the cooling includes introducing a liquid first concentrate including the oligomerization reaction by-product to a cooling device and then cooling the first concentrate to convert the concentrate into a gel phase, and crushing the gelled first concentrate.

13. The method of claim 12,
wherein further cooling is included after the crushing.

14. The method of claim 1,
wherein in the cooling, the first concentrate is cooled to 10°C to 80°C.

15. The method of claim 1,
wherein in the compressing, the cooled first concentrate is compressed at 10°C to 80°C.

## Patentansprüche

1. Verfahren zur Verarbeitung eines Oligomerisationsreaktionsnebenprodukts, umfassend:
in einem Oligomerherstellungsverfahren zur Oligomerisierung eines Monomers und einem Lösungsmittel, um ein Reaktionsprodukt herzustellen,
Einführen eines flüssigen Polymerabfalls in einen Verdampfer, wobei der flüssige Polymerabfall ein Rückstand ist, der nach der Gewinnung von Oligomeren niedriger als C10 und einem nicht-umgesetzten Monomer aus dem Reaktionsprodukt zurückbleibt, und dann Verdampfen einer Fraktion, die C10- oder höhere Oligomere umfasst, um ein erstes Konzentrat zu erhalten, das ein Oligomerisationsreaktionsnebenprodukt umfasst,
Abkühlen des ersten Konzentrats, das die Oligomerisationsreaktionsnebenprodukte umfasst, indem es in eine Kühlvorrichtung eingeführt wird, und
Komprimieren des abgekühlten ersten Konzentrats, um einen zweiten konzentrierten festen Polymerabfall zu erhalten.

2. Verfahren nach Anspruch 1,
wobei in dem Verdampfer eine Fraktion, die C10- bis C22-Oligomere umfasst, verdampft und als oberer Austrittsstrom aus dem Verdampfer gewonnen wird.

3. Verfahren nach Anspruch 1, wobei bei der zweiten Konzentration eine flüssige Aufschlämmung, die C10- bis C22-Oligomere umfasst, durch Kompression gewonnen wird.

4. Verfahren nach Anspruch 3, ferner umfassend:
das Rückführen der flüssigen Aufschlämmung zum Verdampfer.

5. Verfahren nach Anspruch 3, umfassend:
Gewinnen der Fraktion, die die C10- bis C22-Oligomere umfasst, indem an der flüssigen Aufschlämmung eine physikalische Trennung unter Verwendung eines oder mehrerer Vorrichtungen, ausgewählt aus einem Filter, einem Dekanter und einer Zentrifuge, durchgeführt wird.

6. Verfahren nach Anspruch 1,
wobei das Monomer Ethylen einschließt und
die Oligomere ein lineares α-Olefin einschließen.

7. Verfahren nach Anspruch 1,
wobei das Oligomerisationsreaktionsnebenprodukt ein ultrahochmolekulares Polyethylen einschließt und
das ultrahochmolekulare Polyethylen ein gewichtsmittleres Molekulargewicht (Mw) von 1.000.000 g/mol oder mehr aufweist.

8. Verfahren nach Anspruch 7,
wobei das Oligomerisationsreaktionsnebenprodukt zu 0,1 Gew.-% bis 20 Gew.-%, bezogen auf 100 Gew.-% des in den Verdampfer eingeführten flüssigen Polymerabfalls, enthalten ist und
das ultrahochmolekulare Polyethylen zu 1 Gew.-% bis 50 Gew.-%, bezogen auf 100 Gew.-% des Oligomerisationsreaktionsnebenprodukts, enthalten ist.

9. Verfahren nach Anspruch 1,
wobei ein Gesamtfeststoffgehalt (TSC) des Oligomerisationsreaktionsnebenprodukts in dem ersten Konzentrat 10 Gew.-% bis 30 Gew.-% beträgt und
ein Gesamtfeststoffgehalt (TSC) des Oligomerisationsreaktionsnebenprodukts in dem zweiten konzentrierten festen Polymerabfall 25 Gew.-% bis 95 Gew.-% beträgt.

10. Verfahren nach Anspruch 1,
wobei der in den Verdampfer eingeführte flüssige Polymerabfall erhalten wird, indem ein Monomerstrom und ein Lösungsmittelstrom einem Reaktor zugeführt werden, um eine Oligomerisationsreaktion durchzuführen, um das Reaktionsprodukt herzustellen, und ein Reaktoraustrittsstrom, der das Reaktionsprodukt einschließt, in eine Trennvorrichtung eingeführt wird, um eine Fraktion zu gewinnen, die das nicht-umgesetzte Monomer und die Oligomere niedriger als C10 einschließen.

11. Verfahren nach Anspruch 1,
wobei eine Innentemperatur des Verdampfers 100°C bis 250°C beträgt und ein Innendruck des Verdampfers 1 Torr bis 760 Torr beträgt.

12. Verfahren nach Anspruch 1,
wobei das Abkühlen das Einführen eines ersten flüssigen Konzentrats, das das Oligomerisationsreaktionsnebenprodukt einschließt, in eine Kühlvorrichtung und dann das Abkühlen des ersten Konzentrats zur Umwandlung des Konzentrats in eine Gelphase und das Zerkleinern des gelierten ersten Konzentrats einschließt.

13. Verfahren nach Anspruch 12,
wobei nach dem Zerkleinern ein weiteres Abkühlen eingeschlossen ist.

14. Verfahren nach Anspruch 1,
wobei beim Abkühlen das erste Konzentrat auf 10°C bis 80°C abgekühlt wird.

15. Verfahren nach Anspruch 1,
wobei beim Komprimieren das abgekühlte erste Konzentrat bei 10°C bis 80°C komprimiert wird.

## Revendications

1. Procédé de traitement d'un sous-produit de réaction d'oligomérisation, comprenant :
dans un procédé de préparation d'oligomère consistant à oligomériser un monomère et un solvant pour préparer un produit de réaction,
introduire un déchet polymère liquide dans un évaporateur, dans lequel le déchet polymère liquide est un résidu restant après récupération d'oligomères inférieurs à C10 et d'un monomère n'ayant pas réagi à partir du produit de réaction, puis évaporer une fraction comprenant des oligomères C10 ou supérieurs pour obtenir un premier concentrat comprenant un sous-produit de réaction d'oligomérisation ;
refroidir le premier concentrat comprenant les sous-produits de réaction d'oligomérisation en l'introduisant dans un dispositif de refroidissement ; et
comprimer le premier concentrat refroidi pour obtenir un deuxième déchet polymère solide concentré.

2. Procédé selon la revendication 1,
dans lequel, dans l'évaporateur, une fraction comprenant des oligomères C10 à C22 est évaporée et récupérée en tant que flux de décharge supérieur provenant de l'évaporateur.

3. Procédé selon la revendication 1, dans lequel, dans la deuxième concentration, une suspension liquide comprenant des oligomères C10 à C22 est récupérée par la compression.

4. Procédé selon la revendication 3, comprenant en outre :
faire recirculer la suspension liquide vers l'évaporateur.

5. Procédé selon la revendication 3, comprenant :
récupérer la fraction comprenant les oligomères C10 à C22 en effectuant une séparation physique sur la suspension liquide en utilisant un ou plusieurs dispositifs sélectionnés parmi un filtre, un décanteur et une centrifugeuse.

6. Procédé selon la revendication 1,
dans lequel le monomère inclut de l'éthylène, et
les oligomères incluent une α-oléfine linéaire.

7. Procédé selon la revendication 1,
dans lequel le sous-produit de réaction d'oligomérisation inclut du polyéthylène à poids moléculaire ultra-élevé, et
le polyéthylène à poids moléculaire ultra-élevé présente une masse moléculaire moyenne en poids (Mw) de 1000000 g/mol ou plus.

8. Procédé selon la revendication 7,
dans lequel le sous-produit de réaction d'oligomérisation est inclus à raison de 0,1 % en poids à 20 % en poids sur la base de 100 % en poids du déchet polymère liquide introduit dans l'évaporateur, et
le polyéthylène à poids moléculaire ultra-élevé est inclus à raison de 1 % en poids à 50 % en poids sur la base de 100 % en poids du sous-produit de réaction d'oligomérisation.

9. Procédé selon la revendication 1,
dans lequel une teneur totale en solides (TSC) du sous-produit de réaction d'oligomérisation dans le premier concentrat est de 10 % en poids à 30 % en poids, et
une teneur totale en solides (TSC) du sous-produit de réaction d'oligomérisation dans le deuxième déchet polymère solide concentré est de 25 % en poids à 95 % en poids.

10. Procédé selon la revendication 1,
dans lequel le déchet polymère liquide introduit dans l'évaporateur est obtenu en alimentant un flux de monomère et un flux de solvant dans un réacteur pour effectuer une réaction d'oligomérisation afin de préparer le produit de réaction, et en introduisant un flux de décharge du réacteur incluant le produit de réaction dans un dispositif de séparation pour récupérer une fraction incluant le monomère n'ayant pas réagi et les oligomères inférieurs à C10.

11. Procédé la revendication 1,
dans lequel une température interne de l'évaporateur est de 100 °C à 250 °C, et une pression interne de l'évaporateur est de 1 torr à 760 torr.

12. Procédé selon la revendication 1,
dans lequel le refroidissement inclut l'introduction d'un premier concentrat liquide incluant le sous-produit de réaction d'oligomérisation dans un dispositif de refroidissement puis le refroidissement du premier concentrat pour convertir le concentrat en une phase de gel, et le broyage du premier concentrat gélifié.

13. Procédé selon la revendication 12,
dans lequel un refroidissement supplémentaire est inclus après le broyage.

14. Procédé selon la revendication 1,
dans lequel, dans le refroidissement, le premier concentrat est refroidi à 10 °C à 80 °C.

15. Procédé selon la revendication 1,
dans lequel, dans la compression, le premier concentrat refroidi est comprimé à 10 °C à 80 °C.
